# FASCICULE DE BREVET EUROPEEN

(11) **EP 0 847 753 B1**
(45) Date de publication et mention de la délivrance du brevet: **23.05.2001**
(21) Numéro de dépôt: 97402879.7
(22) Date de dépôt: 28.11.1997
(51) Int. Cl.: A61K 7/48, A61K 7/02

(54) **Composition cosmétique comprenant l'association d'un polymère filmogène et d'une dispersion de cire et utilisation de ladite association**
Kosmetische Zusammensetzung enthaltend die Kombination eines filmbildendes Polymeres und einer Wachs-Dispersion und Verwendung dieser Kombination
Cosmetical composition comprising the combination of a film-forming polymer and a wax dispersion and use of said combination

(30) Priorité: 11.12.1996 FR 9615225
(43) Date de publication de la demande: 17.06.1998
(73) Titulaire: L'OREAL, 75008 Paris (FR)
(72) Inventeur: de la Poterie, Valérie, 77820 Le Chatelet En Brie (FR); Mellul, Myriam, 94240 L'Hay Les Roses (FR); Bara, Isabelle, 75013 Paris (FR)
(74) Mandataire: Lhoste, Catherine

(56) Documents cités:
- EP-A- 0 628 304
- EP-A- 0 655 234
- EP-A- 0 663 202
- US-A- 5 547 659

## Description

La présente invention a trait à une composition cosmétique susceptible d'être appliquée sur la peau, les semi-muqueuses et/ou les muqueuses. Ladite composition comprend en particulier une dispersion aqueuse de particules de polymère filmogène et peut être utilisée en tant que produit de maquillage notamment du visage et du corps humain.

Les compositions à appliquer sur la peau, les semi-muqueuses et/ou les muqueuses telles que les rouges à lèvres et les fonds de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.
Ces compositions, lorsqu'elles sont appliquées sur la peau, les muqueuses ou les semi-muqueuses, présentent toutefois l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau, les semi-muqueuses ou les muqueuses, et la nécessité de renouveler régulièrement son application.
Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. On a encore constaté que les eye-liners pouvaient également couler. Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé dans le document JP-A-61 65809 des compositions de rouge à lèvres liquide 'sans transfert' contenant de 1 à 70% en poids de résine de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).
On connaît également du document EP-A-602905 des rouges à lèvres 'sans transfert' contenant une silicone volatile et une résine de silicone comportant une chaîne estérifiée pendante ayant au moins 12 atomes de carbone. Le film de rouge à lèvres présente notamment l'inconvénient de manquer de confort à l'application, en particulier d'être trop sec.
Ainsi, d'une manière générale, l'association d'huiles volatiles avec certains composés siliconés permet d'obtenir un résultat 'sans transfert' satisfaisant. Toutefois, les films obtenus après application de ces compositions et évaporation des volatils présentent néanmoins l'inconvénient d'être relativement mats, et conduisent ainsi à un maquillage peu brillant.

Il subsistait donc le besoin d'une composition cosmétique qui transfère peu ou pas du tout, c'est-à-dire d'une composition 'sans transfert', tout en possédant de bonnes propriétés cosmétiques. Il a alors été proposé une composition cosmétique comprenant, dans un système polymérique, une dispersion aqueuse de particules de polymère filmogène. Ledit système doit permettre l'obtention d'un film sur le support sur lequel il est déposé, ledit film devant présenter au moins une des caractéristiques physico-chimiques suivantes : une élongation supérieure à environ 200%, et/ou une dureté inférieure à 110, et/ou un module de Young (module d'élasticité) inférieur à 200 Mpa (voir EP-A-0793957 et EP-A-0775483).

Le film obtenu résiste remarquablement au transfert et présente une bonne résistance à l'eau. Toutefois, on a constaté que son démaquillage pouvait, selon la nature du polymère employé, être peu facile lorsque l'on utilisait des démaquillants usuels, et notamment des démaquillants huileux usuels.

On connaît, entre autre, du document EP-A 652 234 des compositions huiles-dans-eau contenant une dispersion de polymères et des cires.

La présente invention a pour but de proposer une composition cosmétique qui permet d'obtenir un film de très bonne tenue, qui ne transfère pas et ne tache pas un support avec lequel il serait en contact, et qui ne migre pas au cours du temps, tout en possédant de bonnes propriétés cosmétiques, et qui soit de surcroît aisément démaquillable à l'aide des produits de démaquillage existant.

Un objet de l'invention est donc une composition cosmétique susceptible d'être appliquée sur la peau, les semi-muqueuses et/ou les muqueuses, comprenant une dispersion aqueuse de particules de polymère filmogène, caractérisée par le fait qu'elle comprend en outre au moins une dispersion aqueuse de cire, les particules de cires ayant des dimensions moyennes inférieures à 1 micromètre.
Un autre objet de l'invention est une composition de maquillage sans transfert susceptible d'être appliquée sur la peau, les semi-muqueuses et/ou les muqueuses comprenant une dispersion aqueuse de particules de polymère filmogène et au moins une dispersion aqueuse de particules de cire de dimension moyennes inférieures à 1 micromètre.

Encore un objet de l'invention est l'utilisation dans une composition susceptible d'être appliquée sur la peau, les semi-muqueuses et/ou les muqueuses, et qui permet l'obtention d'un film de très bonne tenue et/ou qui ne transfère pas et/ou qui ne migre pas et/ou qui ne tache pas et/ou qui est brillant, de l'association d'une dispersion aqueuse de particules de polymère filmogène et d'une dispersion aqueuse de cire, les particules de cires ayant des dimensions moyennes inférieures à 1 micromètre. Un autre objet de l'invention est l'utilisation dans une composition susceptible d'être appliquée sur la peau, les semi-muqueuses et/ou les muqueuses, de l'association d'une dispersion aqueuse de particules de polymère filmogène et d'une dispersion aqueuse de cire, afin d'obtenir un film de très bonne tenue et/ou qui ne transfère pas et/ou qui ne migre pas et/ou qui ne tache pas et/ou qui est brillant.

Ainsi, la demanderesse a mis en évidence que, de manière inattendue et surprenante, en associant à la dispersion aqueuse de polymère filmogène une microdispersion de cire, il était possible d'obtenir un film qui reste flexible et souple, qui adhère bien sur le support, qui est non gras, qui présente des propriétés 'sans transfert' excellentes (pas de transfert du tout même en frottant) tout en alliant un démaquillage facile avec des démaquillants ou huiles démaquillantes classiques.

La composition selon l'invention permet l'obtention d'un film homogène, qui présente une texture légère et reste confortable à porter tout au long de la journée. Le film peut ne pas être collant, tout en étant mou, souple, élastique et flexible sur la peau; il suit les mouvements du support sur lequel il est déposé, sans se craqueler et/ou se décoller. Il adhère notamment parfaitement sur les lèvres du visage.
D'autre part, le film obtenu peut être très brillant, ou plus ou moins mat, selon la nature des constituants de la composition, d'où une gamme plus étendue de produits de maquillage, brillants ou mats, au choix.

La composition selon l'invention est facilement applicable et s'étale aisément et uniformément sur la peau, les semi-muqueuses et les muqueuses, en particulier sur les lèvres du visage. La composition selon l'invention trouve notamment une application particulièrement intéressante dans le domaine du soin et/ou du maquillage de la peau, des muqueuses et/ou des semi-muqueuses. On entend notamment par muqueuse, la partie interne de la paupière inférieure; parmi les semi-muqueuses, on entend plus particulièrement les lèvres du visage.
Ainsi, la composition selon l'invention trouve une application préférée dans le domaine des produits de maquillage des lèvres du visage, notamment en tant que rouge à lèvres. Elle trouve également une autre application avantageuse dans le domaine des eye-liners, des fonds de teint ou des fards à joues ou à paupières.

La composition selon l'invention comprend donc une dispersion aqueuse de particules de polymère filmogène. Parmi les polymères filmogènes utilisables dans la composition selon la présente invention, on peut citer les polymères synthétiques, de type polycondensat ou de type radicalaire, les polymères d'origine naturelle, et leurs mélanges.

On peut ainsi citer, parmi les polycondensats, les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne peut être, par exemple, un copolymère polyuréthanne, poly-urée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.
Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.
On peut également citer les polyesters, les polyesters amides, les polyesters à chaîne grasse, les polyamides, et les résines époxyesters.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylène diamine, l'hexaméthylènediamine, la méta- ou para-phénylène diamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.

Les polyesters à chaîne grasse peuvent être obtenus par l'utilisation de diols à chaîne grasse lors de la polycondensation.
Les résines époxyesters peuvent être obtenues par polycondensation d'acides gras avec un condensât aux extrémités α, ω - diépoxy.

Les polymères de type radicalaires peuvent être notamment des polymères, ou des copolymères, acryliques et/ou vinyliques. On utilise de préférence des polymères radicalaires anioniques.
Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.
Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.
Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.
On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.
On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur base de ses connaissances générales, notamment par polymérisation en émulsion ou par mise en dispersion du polymère préalablement formé.

Afin d'améliorer le caractère filmogène d'un polymère, par exemple en abaissant sa température de transition vitreuse, il est possible d'ajouter à la dispersion un agent de coalescence, qui peut être choisi parmi les agents de coalescence connus. Dans la présente description, on entend par 'dispersion de polymère filmogène', une dispersion susceptible de former un film, comprenant ou ne comprenant pas d'agent de coalescence.

La teneur en matière sèche desdites dispersions aqueuses selon la présente invention peut être de l'ordre de 5-60% en poids, et de préférence 30-40%.
La taille des particules de polymères en dispersion aqueuse peut être comprise entre 10-500 nm, et est de préférence comprise entre 20 et 150 nm, ce qui permet d'obtenir un film ayant une brillance remarquable.

Il est possible d'ajouter à la composition selon l'invention, un ou plusieurs polymères hydrosolubles connus, en particulier si l'on souhaite une élimination plus ou moins sensible du film, à l'eau. La quantité de polymère hydrosoluble peut être choisie par l'homme du métier sur base de ses connaissances générales, de manière à obtenir un film ayant les propriétés mécaniques souhaitées, tout en conservant à la composition des propriétés cosmétiquement acceptables.
Parmi les polymères hydrosolubles susceptibles d'être utilisés, on peut citer les dérivés de protéines d'origine animale ou végétale et plus particulièrement les hydrolysats de kératine et les kératines sulfoniques, la polyvinylpyrrolidone, les copolymères vinyliques tels que le copolymère de l'éther méthylvinylique et de l'anhydride maléique ou le copolymère de l'acétate de vinyle et de l'acide crotonique, les glycoaminoglycanes, l'acide hyaluronique et ses dérivés, les polymères acryliques, les polysaccharides, les polymères cellulosiques et leurs dérivés.

La composition peut également comprendre au moins un agent plastifiant, hydrophile ou hydrophobe, choisi pour sa compatibilité avec le ou les polymères et en quantité telle qu'elle ne nuit pas à la sensibilité du film à l'eau. Ledit agent plastifiant peut être choisi parmi tous les composés connus de l'homme du métier comme étant susceptibles de remplir la fonction recherchée. Cet agent peut être hydrosoluble ou insoluble dans l'eau et peut éventuellement se présenter sous forme de dispersion aqueuse.
En particulier, on peut citer, seuls ou en mélange, les plastifiants usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
La quantité d'agent plastifiant peut être choisie par l'homme du métier sur base de ses connaissances générales, de manière à obtenir un film ayant les propriétés mécaniques souhaitées, tout en conservant à la composition des propriétés cosmétiquement acceptables.

Selon l'invention, le système polymérique, comprenant la dispersion aqueuse de polymère, les éventuels agents de coalescence et les éventuels plastifiants, doit permettre l'obtention d'un film sur le support sur lequel il est déposé.
Ledit film vérifie de préférence, dans les conditions de mesure définies avant les exemples, au moins une des conditions physico-chimiques suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110, de préférence inférieure à 70, plus préférentiellement inférieure à 55.

La composition peut comprendre 1-60% en poids, de préférence 5-30% en poids de matière sèche de polymères filmogènes qui seront mis en dispersion aqueuse.

La composition selon l'invention comprend par ailleurs une dispersion aqueuse de cire, de préférence une microdispersion aqueuse de cire. On entend par microdispersion de cire, une dispersion aqueuse de particules de cire, dans laquelle la taille desdites particules de cire est inférieure ou égale à environ 1 micron.
Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une émulsion du type huile-dans-eau. Au refroidissement, on obtient une microdispersion stable de particules colloïdales solides de cire.

Les particules de la microdispersion de cire ont de préférence des dimensions moyennes inférieures à 1 micron, de préférence inférieures à 0,5 micron.
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides, à température ambiante d'origine animale, végétale, minérale ou de synthèse et leurs mélanges. De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40, mesuré selon la norme américaine ASTM D 5 ou selon la norme française NFT 004. Les cires sont des substances insolubles dans l'eau et solubles dans les huiles. Elles contribuent à la formation d'un dépôt filmogène, sans pour autant former seules, un film isolable.

On peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone.

Il est également possible d'utiliser des mélanges commerciaux de cires auto-émulsionnables contenant une cire et des tensioactifs. On peut utiliser par exemple la cire commercialisée sous la dénomination 'Cire Auto Lustrante OFR' par Tiscco, qui contient des cires de Carnauba et de paraffine en association avec des tensioactifs non ioniques, ou la cire auto-émulsionnable commercialisée sous la dénomination 'Cerax A.O. 28/B' par La Ceresine, qui contient de la cire d'Alfa en association avec un tensioactif non ionique. Ces mélanges commerciaux permettent de préparer des microdispersions de cires par simple addition d'eau.
On peut encore citer les produits 'Aquacer' de Byk Cera, et notamment : le mélange de cires synthétiques et naturelles avec émulsionnant anionique (Aquacer 520), la cire de polyéthylène avec émulsionnant non ionique ( Aquacer 514 ou 513), la cire polymérique avec émulsionnant anionique (Aquacer 511). On peut également citer le mélange de cires de polyéthylène et de paraffine avec émulsionnant non ionique ' Jonwax 120' de Johnson Polymer.

La composition selon l'invention peut comprendre, de préférence, de 0,1 à 50% en poids de matière sèche de cire, notamment 1 à 30% en poids. Elle peut également comprendre une quantité suffisante de tensioactif pour permettre d'obtenir une microdispersion de cire, ainsi qu'une composition finale, stable. Notamment, elle peut comprendre 0,01 à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

Il est possible d'introduire en outre dans la phase cireuse microparticulaire des ingrédients actifs liposolubles, tels que des filtres U.V., des vitamines liposolubles, des actifs cosmétiques liposolubles.

Les compositions selon l'invention peuvent se présenter sous différentes formes et en particulier sous forme d'émulsions huile-dans-eau ou eau-dans-huile ou sous forme de dispersions aqueuses.

Selon une forme préférée de réalisation, elles se présentent sous forme d'émulsions huile-dans-eau, qui peut comprendre au moins un tensioactif notamment anionique ou non ionique, en une proportion comprise entre 2 et 30 % en poids par rapport au poids total de la composition.

La composition peut en outre comprendre une matière colorante utilisée de manière usuelle dans le domaine de la cosmétique et du maquillage, telle qu'un colorant hydrosoluble et/ou un pigment.
Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique.
On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, et leurs mélanges.

On peut également ajouter dans la composition selon l'invention tout additif connu tel que des agents épaississants, par exemple des argiles, des gommes, des silices, les dérivés cellulosiques, un polymère synthétique tel qu'un polymère acrylique ou un polymère associatif de type polyuréthanne; une gomme naturelle telle que la gomme xanthane; des agents d'étalement; des dispersants; des conservateurs; des agents antimousses; des agents mouillants; des filtres UV; des parfums; des charges; des actifs cosmétiques; des hydratants; des vitamines et leurs dérivés; des matières biologiques et leurs dérivés.
Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Cette composition doit bien entendu être apte à se déposer sur un support tel que la peau, les muqueuses et/ou les semi-muqueuses.

La composition selon l'invention peut se présenter sous forme fluide, gélifiée, semi-solide, pâte souple, voire solide telle que de stick ou bâton.
Elle trouve en particulier une application pour la préparation ou en tant que produit de maquillage, notamment en tant que rouge à lèvres, fond de teint, fard à joues ou fard à paupières, ou encore eye-liner. On peut également envisager une application dans le domaine des compositions de soin, des compositions solaires ou autobronzantes, à appliquer sur la peau, les semi-muqueuses et/ou les muqueuses.

Le démaquillage des compositions selon l'invention se fait avec des démaquillants classiques et/ou des huiles démaquillantes.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns.
Les échantillons mesurés ont une largeur de 5 mm et une épaisseur de 100 microns. La distance entre les mors est de 25 mm. La vitesse de traction est de 1000 mm par minute.
Dans le cadre de l'exemple 1, on effectue également une mesure pour une vitesse de traction de 10 mm par minute.

### Exemple 1

On a préparé des compositions aqueuses comprenant :
- 4,8% de pâte pigmentaire qui comprend 10-16% en poids de glycérine, de manière à obtenir 2% en poids de pigments dans la composition, et
- les composés suivants :
   composition A : microdispersion de cire de polyéthylène (33% de matière sèche (MS) dans la composition finale AQUACER 513 de Byk Cera
   composition B : microdispersion de cire polymérique (23% MS) AQUACER 511
   composition C : microdispersion de cire de polyéthylène (33% MS) + polymère hydrosoluble (PVP à 10% MS)
   composition D : dispersion aqueuse de polymère acrylique (47% MS) LUHYDRAN LR8763 de BASF
   composition E : dispersion aqueuse de polymère acrylique (42% MS) + microdispersion de cire polymérique (11% MS) AQUACER 511
   composition F : dispersion aqueuse de polymère acrylique (20% MS) + microdispersion de cire polymérique (11% MS) AQUACER 511

On applique les compositions sur les lèvres de 10 personnes qui évaluent de manière sensorielle les critères suivants : transfert, démaquillage à l'eau froide et à l'huile.

On obtient les résultats suivants :

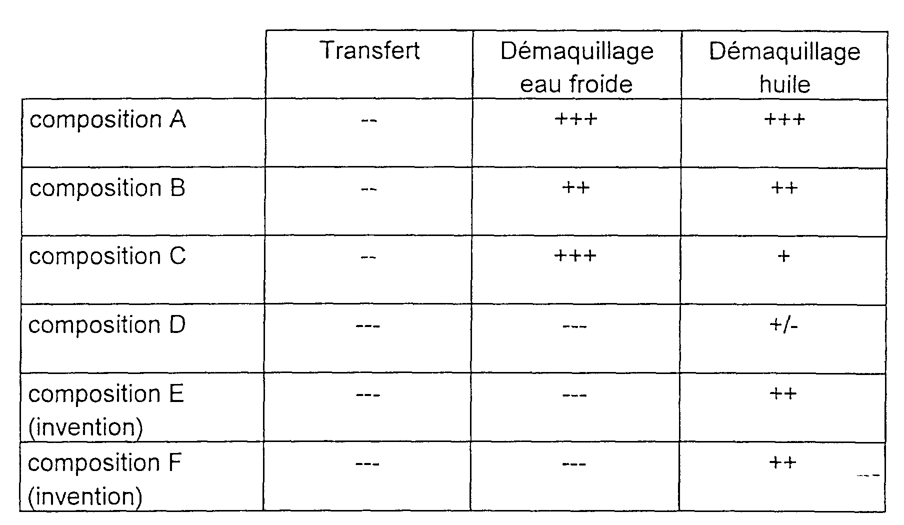

Ainsi, on a constaté que les compositions selon l'invention présentent une bonne tenue à l'eau froide, mais se démaquillent à l'aide d'un démaquillant huileux classique.
De plus, on a constaté que les compositions selon l'invention présentaient une bonne brillance lorsqu'elles étaient appliquées sur les lèvres.

### Exemple 2

On prépare une composition comprend les constituants suivants :
- Dispersion aqueuse de polyuréthanne (40% MS) Sancure 861 de Sanncor 75%
- Microdispersion de cire polymérique (25% MS) Aquacer 27RC601 de Byk Cera 20%
- Pigment 2%
- Glycérine 1,25%
- Eau qsp 100%

On obtient une composition de rouge à lèvres qui s'applique aisément sur les lèvres, et forme un film brillant, qui ne transfère pas et se démaquille aisément.

### Exemple 3

On prépare une composition comprend les constituants suivants :
- Dispersion aqueuse de polymère acrylique (47% MS) LUHYDRAN LR8763 de BASF 64%
- Microdispersion de cire polymérique (25% MS) Aquacer 511 de Byk Cera 20%
- Pigment 2%
- Glycérine 1,25%
- Eau qsp 100%

On obtient une composition de rouge à lèvres qui s'applique aisément sur les lèvres, et forme un film brillant, qui ne transfère pas et se démaquille.

## Revendications

1. Composition cosmétique susceptible d'être appliquée sur la peau, les semi-muqueuses et/ou les muqueuses, comprenant une dispersion aqueuse de particules de polymère filmogène, caractérisée par le fait qu'elle comprend en outre au moins une dispersion aqueuse de particules de cire de dimensions moyennes inférieures à 1 micromètre.

2. Composition selon la revendication 1, dans laquelle le polymère filmogène est choisi parmi les polyuréthannes anioniques, cationiques, non ioniques ou amphotères; les polyuréthannes-acryliques; les polyuréthannes-polyvinylpirrolidones; les polyester-polyuréthannes; les polyéther-polyuréthannes; les polyurées; les polyurée/polyuréthannes; les polyesters, les polyesters amides, les polyesters à chaîne grasse, les polyamides, les résines époxyesters; les polymères ou copolymères acryliques et/ou vinyliques; les copolymères acryliques/silicones; les copolymères nitrocellulose/acryliques; les polymères d'origine naturelle, éventuellement modifiés; les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes; leurs mélanges.

3. Composition selon l'une des revendications précédentes, dans laquelle la taille des particules de polymère filmogène en dispersion aqueuse est comprise entre 10-500 nm, de préférence comprise entre 20 et 150 nm.

4. Composition selon l'une des revendications précédentes, dans laquelle la dispersion aqueuse de polymère filmogène permet l'obtention d'un film ayant au moins une des conditions physico-chimiques suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110, de préférence inférieure à 70, plus préférentiellement inférieure à 55.

5. Composition selon l'une des revendications précédentes, comprenant 1-60% en poids, de préférence 5-30% en poids de matière sèche de polymères filmogènes qui seront mis en dispersion aqueuse.

6. Composition selon l'une des revendications précédentes, comprenant en outre au moins un agent plastifiant tel que les glycols et leurs dérivés, les esters de glycérol, les dérivés de propylène glycol, des esters d'acides notamment carboxyliques, des dérivés oxyéthylénés; leur mélange.

7. Composition selon l'une des revendications précédentes, dans laquelle les particules de la dispersion de cire ont des dimensions moyennes inférieures à 0,5 micromètre.

8. Composition selon l'une des revendications précédentes, dans laquelle la cire est choisie parmi les cires ayant un point de fusion supérieur à 45°C environ et/ou un indice de pénétration de l'aiguille à 25°C compris entre 3 et 40.

9. Composition selon l'une des revendications précédentes, dans laquelle la cire est choisie parmi la cire d'abeilles, la cire de lanoline, les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch; les copolymères cireux ainsi que leurs esters; les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

10. Composition selon l'une des revendications précédentes, comprenant 0,1 à 50% en poids de matière sèche de cire, notamment 1 à 30% en poids.

11. Composition selon l'une des revendications précédentes, dans laquelle les particules de la dispersion de cire comprennent en outre des additifs gras huileux et/ou pâteux, un tensioactif et/ou un additif/actif liposoluble usuel.

12. Composition selon l'une des revendications précédentes, comprenant en outre au moins un tensioactif.

13. Composition selon l'une des revendications précédentes, comprenant en outre au moins un polymère hydrosoluble, tel que les dérivés de protéines d'origine animale ou végétale, plus particulièrement les hydrolysats de kératine et les kératines sulfoniques, la polyvinylpyrrolidone, les copolymères vinyliques tels que le copolymère de l'éther méthylvinylique et de l'anhydride maléique ou le copolymère de l'acétate de vinyle et de l'acide crotonique, les glycoaminoglycanes, l'acide hyaluronique et ses dérivés, les polymères acryliques, les polysaccharides, les polymères cellulosiques et leurs dérivés.

14. Composition selon l'une des revendications précédentes, comprenant en outre une matière colorante.

15. Composition selon la revendication 14, dans laquelle la matière colorant est choisie parmi les colorants hydrosolubles et/ou les pigments.

16. Composition selon l'une des revendications précédentes, se présentant sous forme d'émulsion huile-dans-eau ou eau-dans-huile ou sous forme de dispersion aqueuse.

17. Composition selon l'une des revendications précédentes, se présentant sous la forme d'un produit de maquillage tel qu'un rouge à lèvres, un fond de teint, un fard à joues ou fard à paupières, un eye-liner; d'une composition dé soin; d'une composition solaire ou autobronzante.

18. Composition selon l'une des revendications précédentes, permettant après application l'obtention d'un film de très bonne tenue et/ou qui ne transfère pas et/ou qui ne migre pas et/ou qui ne tache pas et/ou qui est brillant.

19. Composition de maquillage sans transfert susceptible d'être appliquée sur la peau, les semi-muqueuses et/ou les muqueuses comprenant une dispersion aqueuse de particules de polymère filmogène et au moins une dispersion aqueuse de particules de cire de dimensions moyennes inférieures à 1 micromètre.

20. Utilisation dans une composition susceptible d'être appliquée sur la peau, les semi-muqueuses et/ou les muqueuses, et qui permet l'obtention d'un film de très bonne tenue et/ou qui ne transfère pas et/ou qui ne migre pas et/ou qui ne tache pas et/ou qui est brillant, de l'association d'une dispersion aqueuse de particules de polymère filmogène et d'une dispersion aqueuse de particules de cire de dimensions moyennes inférieures à 1 micromètre.

21. Utilisation dans une composition susceptible d'être appliquée sur la peau, les semi-muqueuses et/ou les muqueuses, de l'association d'une dispersion aqueuse de particules de polymère filmogène et d'une dispersion aqueuse de cire, afin d'obtenir un film de très bonne tenue et/ou qui ne transfère pas et/ou qui ne migre pas et/ou qui ne tache pas et/ou qui est brillant, les particules de cires ayant des dimensions moyennes inférieures à 1 micromètre.

22. Utilisation selon l'une des revendications 20 à 21, dans laquelle la dispersion aqueuse de polymère filmogène permet l'obtention d'un film ayant au moins une des conditions physico-chimiques suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110, de préférence inférieure à 70, plus préférentiellement inférieure à 55.

23. Utilisation selon l'une des revendications 20 à 22, dans laquelle les particules de la dispersion de cire ont des dimensions moyennes inférieures à 0,5 micron.

24. Utilisation selon l'une des revendications 20 à 23, dans laquelle la composition comprend en outre une matière colorante qui peut être choisie parmi les colorants hydrosolubles et/ou les pigments.

25. Utilisation selon l'une des revendications 20 à 24, dans une composition se présentant sous la forme d'un produit de maquillage tel qu'un rouge à lèvres, un fond de teint, un fard à joues ou fard à paupières, un eye-liner; d'une composition de soin; d'une composition solaire ou autobronzante.

## Claims

1. Cosmetic composition which may be applied to the skin, to semi-mucous membranes and/or to mucous membranes, including an aqueous dispersion of film-forming polymer particles, characterized in that it also includes at least one aqueous dispersion of wax particles with average sizes of less than 1 micrometre.

2. Composition according to Claim 1, in which the film-forming polymer is chosen from anionic, cationic, nonionic or amphoteric polyurethanes; polyurethane-acrylics; polyurethane-polyvinylpirrolidones; polyester-polyurethanes; polyether-polyurethanes; polyureas; polyurea-polyurethanes; polyesters, polyester amides, fatty-chain polyesters, polyamides, epoxy ester resins, acrylic and/or vinyl polymers or copolymers; acrylic/ silicone copolymers; nitrocellulose/acrylic copolymers; optionally modified polymers of natural origin; the polymers resulting from the radical polymerization of one or more radical monomers inside and/or partially at the surface of preexisting particles of at least one polymer chosen from the group consisting of polyurethanes, polyureas, polyesters, polyester amides and/or alkyds; mixtures thereof.

3. Composition according to either of the preceding claims, in which the size of the film-forming polymer particles in aqueous dispersion is between 10-500 nm, preferably between 20 and 150 nm.

4. Composition according to one of the preceding claims, in which the aqueous dispersion of film-forming polymer makes it possible to obtain a film having at least one of the following physicochemical conditions:
· a Young's modulus of less than about 200 MPa, preferably less than about 100 MPa and preferably less than 80 MPa, and/or
· an elongation of greater than about 200%, and preferably greater than 300%, and/or
· a hardness of less than 110, preferably less than 70 and more preferably less than 55.

5. Composition according to one of the preceding claims, comprising 1-60% by weight, preferably 5-30% by weight, of film-forming polymer solids which will be placed in aqueous dispersion.

6. Composition according to one of the preceding claims, also comprising at least one plasticizer such as glycols and derivatives thereof, glycerol esters, propylene glycol derivatives, acid esters, in particular carboxylic acid esters, oxyethylenated derivatives; a mixture thereof.

7. Composition according to one of the preceding claims, in which the particles of the wax dispersion have average sizes of less than 0.5 micrometre.

8. Composition according to one of the preceding claims, in which the wax is chosen from waxes having a melting point of greater than approximately 45°C and/or a needle-penetration index at 25°C of between 3 and 40.

9. Composition according to one of the preceding claims, in which the wax is chosen from beeswax, lanolin wax and Chinese insect waxes; rice wax, carnauba wax, candelilla wax, ouricurry wax, alfalfa wax, cork fibre wax, sugarcane wax, Japan wax, sumac wax, montan wax, microcrystalline waxes, paraffins, ozokerite; polyethylene waxes, the waxes obtained by Fisher-Tropsch synthesis; waxy copolymers and esters thereof; the waxes obtained by catalytic hydrogenation of animal or vegetable oils having linear or branched C8-C32 fatty chains; hydrogenated jojoba oil, hydrogenated sunflower oil, hydrogenated castor oil, hydrogenated coconut oil, hydrogenated lanolin oil; silicone waxes; mixtures thereof.

10. Composition according to one of the previous claims, comprising 0.1 to 50% by weight of wax solids, in particular 1 to 30% by weight.

11. Composition according to one of the preceding claims, in which the particles of the wax dispersion also comprise oily and/or pasty fatty additives, a surfactant and/or a common liposoluble additive/active agent.

12. Composition according to one of the preceding claims, also comprising at least one surfactant.

13. Composition according to one of the preceding claims, also comprising at least one water-soluble polymer, such as protein derivatives of animal or plant origin, more particularly keratin hydrolysates and sulphonic keratins, polyvinylpyrrolidone, vinyl copolymers such as the copolymer of methyl vinyl ether and of maleic anhydride or the copolymer of vinyl acetate and of crotonic acid, glycoaminoglycans, hyaluronic acid and derivatives thereof, acrylic polymers, polysaccharides, cellulose polymers and derivatives thereof.

14. Composition according to one of the preceding claims, also comprising a dyestuff.

15. Composition according to Claim 14, in which the dyestuff is chosen from water-soluble dyes and/or pigments.

16. Composition according to one of the preceding claims, in the form of an oil-in-water or water-in-oil emulsion or in the form of an aqueous dispersion.

17. Composition according to one of the preceding claims, in the form of a make-up product such as a lipstick, a foundation, a blusher, an eyeshadow or an eye-liner; a care composition; an antisun composition or a self-tanning composition.

18. Composition according to one of the preceding claims, which makes it possible to obtain, after it has been applied, a film with very good staying power and/or one which does not transfer and/or one which does not migrate and/or one which does not stain and/or one which is glossy.

19. Transfer-resistant make-up composition which can be applied to the skin, semi-mucous membranes and/or mucous membranes, comprising an aqueous dispersion of film-forming polymer particles and at least one aqueous dispersion of wax particles with average sizes of less than 1 micrometre.

20. Use, in a composition which can be applied to the skin, semi-mucous membranes and/or mucous membranes and which allows a film to be produced which has very good staying power and/or which does not transfer and/or which does not migrate and/or which does not stain and/or which is glossy, of a combination of an aqueous dispersion of film-forming polymer particles and an aqueous dispersion of wax particles with average sizes of less than 1 micrometre.

21. Use, in a composition which can be applied to the skin, semi-mucous membranes and/or mucous membranes, of a combination of an aqueous dispersion of film-forming polymer particles and an aqueous wax dispersion, in order to obtain a film which has very good staying power and/or which does not transfer and/or which does not migrate and/or which does not stain and/or which is glossy, the wax particles having average sizes of less than 1 micrometre.

22. Use according to either of Claims 20 and 21, in which the aqueous dispersion of film-forming polymer makes it possible to obtain a film having at least one of the following physicochemical conditions:
· a Young's modulus of less than about 200 MPa, preferably less than about 100 MPa and preferably less than 80 MPa, and/or
· an elongation of greater than about 200%, and preferably greater than 300%, and/or
· a hardness of less than 110, preferably less than 70 and more preferably less than 55.

23. Use according to one of Claims 20 to 22, in which the particles of the wax dispersion have average sizes of less than 0.5 micron.

24. Use according to one of Claims 20 to 23, in which the composition also comprises a dyestuff which may be chosen from water-soluble dyes and/or pigments.

25. Use according to one of Claims 20 to 24, in a composition in the form of a make-up product such as a lipstick, a foundation, a blusher, an eyeshadow or an eye-liner; a care composition; an antisun composition or a self-tanning composition.

## Patentansprüche

1. Kosmetische Zusammensetzung, die auf die Haut, die Semischleimhäute und/oder die Schleimhäute aufgetragen werden kann und die eine wäßrige Dispersion von Partikeln eines filmbildenden Polymers enthält, dadurch gekennzeichnet, daß sie ferner mindestens eine wäßrige Dispersion von Wachspartikeln enthält, die eine mittlere Größe unter 1 µm aufweisen.

2. Zusammensetzung nach Anspruch 1, wobei das filmbildende Polymer ausgewählt ist unter den anionischen, kationischen, nichtionischen oder amphoteren Polyurethanen, Polyurethan-Acryl-Polymeren, Polyurethan-Polyvinylpyrrolidonen, Polyester-Polyurethanen, Polyether-Polyurethanen, Polyharnstoffen, Polyharnstoff/Polyurethanen, Polyestern, Polyesteramiden, Polyestern mit Fettkette, Polyamiden, Epoxyesterharzen, Acryl- und/oder Vinylpolymeren, Acryl- und/oder Vinylcopolymeren, Acryl/Siliconcopolymeren, Nitrocellulose/Acrylcopolymeren, Polymeren natürlichen Ursprungs, die gegebenenfalls modifiziert sind, Polymeren, die aus der radikalischen Polymerisation eines oder mehrerer radikalischer Monomere im Innern und/oder zum Teil an der Oberfläche von vorab gebildeten Partikeln mindestens eines Polymers stammen, das unter den Polyurethanen, Polyharnstoffen, Polyestern, Polyesteramiden und/oder Alkydharzen ausgewählt ist, und deren Gemischen.

3. Zusammensetzung nach einem der vorhergehenden Ansprüche, wobei die Partikel des filmbildenden Polymers in wäßriger Dispersion eine Größe im Bereich von 10 bis 500 nm und vorzugsweise im Bereich von 20 bis 150 nm aufweisen.

4. Zusammensetzung nach einem der vorhergehenden Ansprüche, wobei durch die wäßrige Dispersion des filmbildenden Polymers ein Film erhalten werden kann, der mindestens eine der folgenden physikalisch-chemischen Bedingungen erfüllt:
- ein Young-Modul unter etwa 200 MPa, vorzugsweise unter etwa 100 MPa und noch bevorzugter unter 80 MPa, und/oder
- eine Dehnung über etwa 200 % und vorzugsweise über 300 %, und/oder
- eine Härte unter 110, vorzugsweise unter 70 und noch bevorzugter unter 55.

5. Zusammensetzung nach einem der vorhergehenden Ansprüche, die 1 bis 60 Gew.-% und vorzugsweise 5 bis 30 Gew.-% Trockensubstanz filmbildender Polymere enthält, die in Form einer wäßrigen Dispersion dispergiert werden.

6. Zusammensetzung nach einem der vorhergehenden Ansprüche, die ferner mindestens einen Weichmacher enthält, der unter den Glykolen und ihren Derivaten, Glycerinestern, Propylenglykolderivaten, Estern von Säuren, insbesondere von Carbonsäuren, ethoxylierten Derivaten und deren Gemischen ausgewählt ist.

7. Zusammensetzung nach einem der vorhergehenden Ansprüche, wobei die Wachspartikel der Dispersion eine mittlere Größe unter 0,5 µm aufweisen.

8. Zusammensetzung nach einem der vorhergehenden Ansprüche, wobei das Wachs unter den Wachsen ausgewählt ist, die einen Schmelzpunkt über etwa 45 °C und/oder eine Nadelpenetrationszahl bei 25 °C im Bereich von 3 bis 40 aufweisen.

9. Zusammensetzung nach einem der vorhergehenden Ansprüche, wobei das Wachs ausgewählt ist unter: Bienenwachs, Lanolinwachs, Chinawachsen; Reiswachs, Carnaubawachs, Candellilawachs, Ouricurywachs, Alfawachs, Korkfaser-Wachs, Zuckerrohr-Wachs, Japanwachs und Sumachwachs; Montanwachs, mikrokrisatllinen Wachsen, Paraffinen und Ozokerit; Polyethylenwachsen, durch Fischer-Tropsch-Synthese hergestellten Wachsen und wachsigen Copolymeren sowie deren Estern; Wachsen, die durch katalytische Hydrierung von tierischen oder pflanzlichen Ölen mit geradkettigen oder verzweigten C₈₋₃₂-Fettketten hergestellt sind; hydriertem Jojobaöl, hydriertem Sonnenblumenöl, hydriertem Ricinusöl, hydriertem Kopraöl und hydriertem Lanolinöl; Siliconwachsen und deren Gemischen.

10. Zusammensetzung nach einem der vorhergehenden Ansprüche, die 0,1 bis 50 Gew.-% und insbesondere 1 bis 30 Gew.-% Trockensubstanz des Wachses enthält.

11. Zusammensetzung nach einem der vorhergehenden Ansprüche, wobei die Partikel der Wachsdispersion ferner ölige und/oder pastöse Zusatzstoffe, einen grenzflächenaktiven Stoff und/oder einen üblichen fettlöslichen Hilfsstoff/Wirkstoff enthalten.

12. Zusammensetzung nach einem der vorhergehenden Ansprüche, die ferner mindestens einen grenzflächenaktiven Stoff enthält.

13. Zusammensetzung nach einem der vorhergehenden Ansprüche, die ferner mindestens ein wasserlösliches Polymer enthält, beispielsweise Proteinderivate tierischen oder pflanzlichen Ursprungs und insbesondere Keratinhydrolysate und Keratinverbindungen mit Sulfonsäuregruppen, Polyvinylpyrrolidon, Vinylcopolymere, wie das Copolymer von Methylvinylether und Maleinsäureanhydrid und das Copolymer von Vinylacetat und Crotonsäure, Glykosaminoglykane, Hyaluronsäure und ihre Derivate, Acrylpolymere, Polysaccharide, Celluloseverbindungen und ihre Derivate.

14. Zusammensetzung nach einem der vorhergehenden Ansprüche, die ferner ein Färbemittel enthält.

15. Zusammensetzung nach Anspruch 14, wobei das Färbemittel unter den wasserlöslichen Farbstoffen und/oder den Pigmenten ausgewählt ist.

16. Zusammensetzung nach einem der vorhergehenden Ansprüche, die in Form einer Öl-in-Wasser- oder Wasser-in-Öl-Emulsion oder in Form einer wäßrigen Dispersion vorliegt.

17. Zusammensetzung nach einem der vorhergehenden Ansprüche, die in Form eines Produkts zum Schminken, wie beispielsweise als Lippenstift, Make-up, Wangenrouge, Lidschatten oder Eyeliner, einer Zusammensetzung zur Pflege, einer Zusammensetzung zum Sonnenschutz oder einer Zusammensetzung zur Selbstbräunung vorliegt.

18. Zusammensetzung nach einem der vorhergehenden Ansprüche, wobei durch die Zusammensetzung nach dem Auftragen ein Film erhalten wird, der sehr gut haftet und/oder sich nicht überträgt und/oder keine Migration zeigt und/oder keine Flecken verursacht und/oder glänzt.

19. Zusammensetzung zum Schminken ohne Transfer, die auf die Haut, die Semischleimhäute und/oder die Schleimhäute aufgetragen werden kann und die eine wäßrige Dispersion von Partikeln eines filmbildenden Polymers und mindestens eine wäßrige Dispersion von Wachspartikeln enthält, die eine mittlere Größe unter 1 µm aufweisen.

20. Verwendung der Kombination einer wäßrigen Dispersion von Partikeln eines filmbildenden Polymers und einer wäßrigen Dispersion von Wachspartikeln, die eine mittlere Größe unter 1 µm aufweisen, in einer Zusammensetzung, die auf die Haut, die Semischleimhäute und/oder die Schleimhäute aufgetragen werden kann und mit der ein Film erhalten werden kann, der sehr gut haftet und/oder sich nicht überträgt und/oder keine Migration zeigt und/oder keine Flecken verursacht und/oder glänzt.

21. Verwendung der Kombination einer wäßrigen Dispersion von Partikeln eines filmbildenden Polymers und einer wäßrigen Dispersion eines Wachses in einer Zusammensetzung, die auf die Haut, die Semischleimhäute und/oder die Schleimhäute aufgetragen werden kann, um einen Film herzustellen, der sehr gut haftet und/oder sich nicht überträgt und/oder keine Migration zeigt und/oder keine Flecken verursacht und/oder Glanz aufweist, wobei die Wachspartikel eine mittlere Größe unter 1 µm aufweisen.

22. Verwendung nach einem der Ansprüche 20 bis 21, wobei durch die wäßrige Dispersion des filmbildenden Polymers ein Film erhalten werden kann, der mindestens eine der folgenden physikalisch-chemischen Bedingungen erfüllt:
- ein Young-Modul unter etwa 200 MPa, vorzugsweise unter etwa 100 MPa und noch bevorzugter unter 80 MPa, und/oder
- eine Dehnung über etwa 200 % und vorzugsweise über 300 %, und/oder
- eine Härte unter 110, vorzugsweise unter 70 und noch bevorzugter unter 55.

23. Verwendung nach einem der Ansprüche 20 bis 22, wobei die Partikel der Wachsdispersion eine mittlere Größe unter 0,5 µm aufweisen.

24. Verwendung nach einem der Ansprüche 20 bis 23, wobei die Zusammensetzung ferner ein Färbemittel enthält, das unter den wasserlöslichen Farbstoffen und/oder den Pigmenten ausgewählt werden kann.

25. Verwendung nach einem der Ansprüche 20 bis 24 in einer Zusammensetzung, die in Form eines Produkts zum Schminken, wie beispielsweise als Lippenstift, Make-up, Wangenrouge, Lidschatten oder Eyeliner, einer Zusammensetzung zur Pflege, einer Zusammensetzung zum Sonnenschutz oder einer Zusammensetzung zur Selbstbräunung vorliegt.
